# EUROPEAN PATENT APPLICATION

(11) **EP 4 765 006 A1**
(43) Date of publication of application: **24.06.2026**
(21) Application number: 24885997.7
(22) Date of filing: 30.08.2024
(51) Int. Cl.: G06Q 50/22, G06Q 50/10, A63B 24/00, A63B 71/06, G16H 20/30, G06N 3/02

(54) **METHOD FOR PROVIDING HEALTH GUIDE, AND ELECTRONIC DEVICE SUPPORTING SAME**

(30) Priority: 30.10.2023 KR 20230146899; 15.12.2023 KR 20230183291
(71) Applicant: Samsung Electronics Co., Ltd., Suwon-si, Gyeonggi-do 16677 (KR)
(72) Inventor: KIM, Jaepil, Suwon-si Gyeonggi-do 16677 (KR); PARK, Jeongmin, Suwon-si Gyeonggi-do 16677 (KR); PARK, Byeongju, Suwon-si Gyeonggi-do 16677 (KR); YEO, Jaeyung, Suwon-si Gyeonggi-do 16677 (KR); LEE, Myeongjin, Suwon-si Gyeonggi-do 16677 (KR); CHO, Sunghwan, Suwon-si Gyeonggi-do 16677 (KR)
(74) Representative: HGF
(86) International application number: PCT/KR2024/013085
(87) International publication number: WO 2025/095317

(57) **Abstract**

This electronic device comprises: a display; a memory for storing a health coaching system composed of first and second sub-coaching models and a manager model; and a processor for controlling the health coaching system so as to output a health guide, wherein the manager model provides a first coaching request query to the first and second sub-coaching models, outputs a health guide including first coaching information, of a first level, acquired from the first sub-coaching model and second coaching information, of the first level, acquired from the second sub-coaching model, selects one of the first and second sub-coaching models as a preferred coaching model on the basis of feedback about the health guide, and, if a second coaching request query is provided to the first and second sub-coaching models, acquires, from the preferred coaching model, third coaching information of a second level, which is higher than the first level and acquires, from the other sub-coaching model, fourth coaching information of the first level.

## Description

### [Technical Field]

Embodiments disclosed in this specification relate to a method for providing a health guide and an electronic device supporting the same.

### [Background Art]

With the development of digital technologies, a variety of electronic devices capable of communicating and processing personal information on the go, such as mobile terminals, electronic organizers, smartphones, tablet PCs, and wearable devices, are being released. Through rapid technological advancements, these electronic devices have evolved from simple voice calls and messaging to include diverse functions such as video calls, electronic organizers, document processing, email, internet access, and photography.

Moreover, the electronic devices may also provide healthcare services. For example, the electronic devices may record measured results of biometric information (e.g., health data) or may record measured results of activity data (e.g., exercise data).

The above information may be provided as a related art for the purpose of enhancing the understanding of this disclosure. No claim or determination is made as to whether any of the foregoing may be applied as a prior art in connection with the disclosure.

### [Disclosure]

### [Technical Problem]

### [Technical Solution]

In general, an electronic device may collect and record biometric information and/or activity information through various sensors. Moreover, the electronic device may provide the collected biometric information and/or activity information along with goal information. For example, the electronic device may guide users toward achieving pre-defined goals by using the biometric information and/or the activity information. For example, messages for encouraging goal achievement, messages for celebrating goal achievement, and actions necessary for goal achievement may be provided as guides.

However, the aforementioned guides are one-dimensional message expressing praise, encouragement, or regret that is provided based on a rule, thereby making it difficult for users of healthcare services to utilize it as meaningful guides.

Accordingly, at least one embodiment of various embodiments may provide an optimized health guide to a user by using coaching information obtained from a plurality of coaching models.

The technical problems to be solved by the disclosure are not limited to the aforementioned problems, and any other technical problems not mentioned herein will be clearly understood from the following description by those skilled in the art to which the disclosure pertains.

According to various embodiments, an electronic device may include a display, a memory that stores a health coaching system including a first sub-coaching model, a second sub-coaching model, and a manager model, and a processor operatively connected with the display and the memory and that is configured to display a health guide through the display by controlling the health coaching system. Under the control of the processor, the manager model may be configured to provide a first coaching request query based on the status information related to a user, to the first sub-coaching model and the second coaching sub model, to obtain the first coaching information of a first level corresponding to the first coaching request query from the first sub-coaching model, to obtain the second coaching information of a first level corresponding to the first coaching request query from the second sub-coaching model, to display the first health guide including the first coaching information and the second coaching information through the display, based on a feedback about the first health guide, to select at least one of the first sub-coaching model and the second sub-coaching model as a preferred coaching model, when providing a second coaching request query based on the status information related to the user to the first sub-coaching model and the second sub-coaching model, to obtain the third coaching information of a second level higher than the first level from the preferred coaching model among the first sub-coaching model and the second sub-coaching model, and to obtain the fourth coaching information of the first level from non-preferred coaching model among the first sub-coaching model and the second sub-coaching model.

According to various embodiments, an operating method of an electronic device may include providing a first coaching request query based on status information related to a user, to a first sub-coaching model and a second coaching sub model, obtaining first coaching information of a first level corresponding to the first coaching request query from the first sub-coaching model, obtaining second coaching information of a first level corresponding to the first coaching request query from the second sub-coaching model, displaying a first health guide including the first coaching information and the second coaching information through a display of the electronic device, based on a feedback about the first health guide, selecting at least one of the first sub-coaching model and the second sub-coaching model as a preferred coaching model, when providing a second coaching request query based on the status information related to the user to the first sub-coaching model and the second sub-coaching model, obtaining third coaching information of a second level higher than the first level from the preferred coaching model and obtaining fourth coaching information of the first level from other sub-coaching model.

### [Advantageous Effects]

According to various embodiments disclosed in this specification, an electronic device may provide an optimized health guide to a user by using coaching information obtained from a plurality of coaching models.

In addition, according to various embodiments disclosed in this specification, an electronic device may obtain meaningful coaching information by inputting various pieces of data into a coaching model preferred by a user among a plurality of coaching models.

Effects obtained in the disclosure are not limited to the above-mentioned effects, and other effects that are not mentioned will be clearly understood by those skilled in the art, to which the disclosure belongs, from the following description.

### [Description of Drawings]

FIG. 1 is a block diagram of an electronic device in a network environment, according to various embodiments.
FIG. 2 is a diagram illustrating a health coaching system, according to various embodiments.
FIG. 3 is a diagram illustrating a coaching operation of a health coaching system, according to various embodiments.
FIG. 4 is a diagram illustrating a health guide, according to various embodiments.
FIG. 5 is a diagram illustrating a preference for a sub-coaching model, according to various embodiments.
FIG. 6 is a diagram illustrating an operation of a manager model assigning a weight to a sub-coaching model, according to various embodiments.
FIG. 7 is a diagram illustrating an operation of assigning a weight to a sub-coaching model, according to various embodiments.
FIG. 8 is a diagram illustrating an operation of a manager model providing a coaching request query, according to various embodiments.
FIG. 9 is a diagram illustrating a coaching query request, according to various embodiments.
FIG. 10 is a diagram illustrating an operation of a manager model providing a coaching request query, according to various embodiments.
FIG. 11 is a diagram illustrating a coaching query request, according to various embodiments.
FIG. 12 is a diagram illustrating an operation of generating a health guide of a manager model, according to various embodiments.
FIG. 13 is a drawing for describing a method of outputting a health guide of a manager model, according to various embodiments.
FIGS. 14A and 14B are diagrams illustrating sequential operations of a health coaching system, according to various embodiments.
FIG. 14C is a diagram illustrating a health guide, according to various embodiments.
FIG. 15 is a diagram illustrating a configuration of a sub-coaching model and a manager model, according to various embodiments.
FIG. 16 is a flowchart illustrating the operation of a health coaching system, according to various embodiments.

### [Mode for Invention]

Hereinafter, various embodiments of the disclosure may be described with reference to accompanying drawings. Accordingly, those of ordinary skill in the art will recognize that modification, equivalent, and/or alternative on the various embodiments described herein may be variously made without departing from the scope and spirit of the disclosure. With regard to description of drawings, similar components may be marked by similar reference numerals.

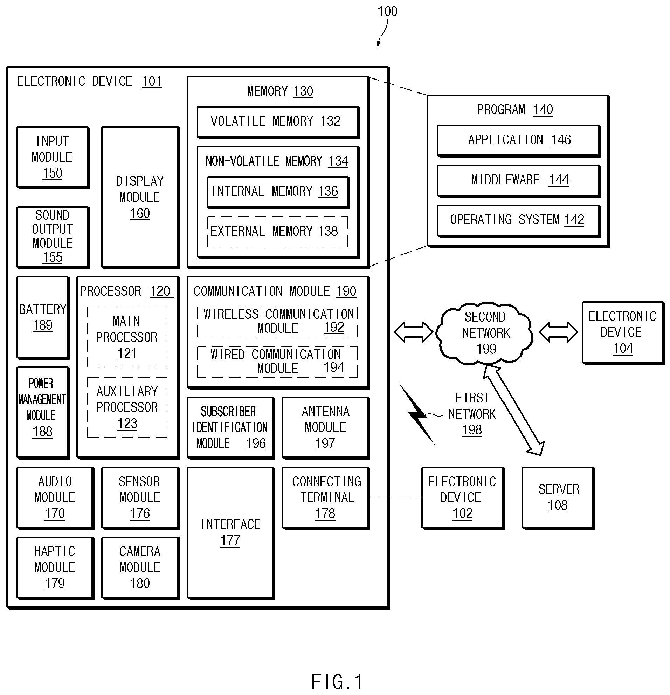

FIG. 1 is a block diagram of an electronic device 101 in a network environment 100, according to various embodiments.

Referring to FIG. 1, the electronic device 101 in the environment information 100 may communicate with an electronic device 102 over a first network 198 (e.g., a short range wireless communication network) or may communicate with at least one of an electronic device 104 or a server 108 over a second network 199 (e.g., a long distance wireless communication network). According to an embodiment, the electronic device 101 may communicate with the electronic device 104 through the server 108. According to an embodiment, the electronic device 101 may include a processor 120, a memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module 196, or an antenna module 197. In any embodiment, the electronic device 101 may not include at least one (e.g., the connecting terminal 178) of the above-described components or may further include one or more other components. In some embodiments, some (e.g., the sensor module 176, the camera module 180, or the antenna module 197) of these components may be integrated into a single component (e.g., the display module 160).

For example, the processor 120 may execute software (e.g., a program 140) to control at least another component (e.g., hardware or software component) of the electronic device 101 connected to the processor 120, and may process and calculate various types of data. According to an embodiment, as at least part of data processing or calculation, the processor 120 may store instructions or data received from other components (e.g., the sensor module 176 or the communication module 190) into a volatile memory 132, may process instructions or data stored in the volatile memory 132, and may store the result data in a nonvolatile memory 134. According to an embodiment, the processor 120 may include a main processor 121 (e.g., a central processing unit or an application processor) and an auxiliary processor 123 (e.g., a graphic processing unit, a neural processing unit (NPU), an image signal processor, a sensor hub processor, or a communication processor) capable of operating independently or together with the main processor. For example, when the electronic device 101 includes the main processor 121 and the auxiliary processor 123, the auxiliary processor 123 may be configured to use less power than the main processor 121 or to be specialized for a specified function. The auxiliary processor 123 may be implemented separately from the main processor 121 or as part of the main processor 121.

For example, the auxiliary processor 123 may control at least part of the functions or states associated with at least one (e.g., the display module 160, the sensor module 176, or the communication module 190) of the components of the electronic device 101, instead of the main processor 121 while the main processor 121 is in an inactive (e.g., sleep) state or together with the main processor 121 while the main processor 121 is in an active (e.g., the execution of an application) state. According to an embodiment, the auxiliary processor 123 (e.g., an image signal processor or a communication processor) may be implemented as a part of operatively associated other components (e.g., the camera module 180 or the communication module 190). According to an embodiment, the auxiliary processor 123 (e.g., a neural network processing unit) may include a hardware structure specialized to process an artificial intelligence model. The artificial intelligence model may be generated through machine learning. For example, the learning may be performed in the electronic device 101, in which an artificial intelligence model is performed, or may be performed through a separate server (e.g., the server 108). For example, the learning algorithm may include supervised learning, unsupervised learning, semi-supervised learning, or reinforcement learning, but is not limited to the above example. The artificial intelligence model may include a plurality of artificial neural network layers. The artificial neural network may be one of a deep neural network (DNN), a convolutional neural network (CNN), a recurrent neural network (RNN), a restricted Boltzmann machine (RBM), a deep belief network (DBN), a bidirectional recurrent deep neural network (BRDNN), a deep Q-network, or a combination of two or more of the networks, but may not be limited to the above-described example. In addition to a hardware structure, additionally or alternatively, the artificial intelligence model may include a software structure.

The memory 130 may store various pieces of data used by at least one component (e.g., the processor 120 or the sensor module 176) of the electronic device 101. For example, data may include software (e.g., the program 140) and input data or output data for instructions associated with the software. The memory 130 may include the volatile memory 132 or the nonvolatile memory 134.

The program 140 may be stored in the memory 130 as software, and may include, for example, an operating system 142, a middleware 144, or an application 146.

The input module 150 may receive instructions or data to be used for the component (e.g., the processor 120) of electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output a sound signal to the outside of the electronic device 101. The sound output module 155 may include, for example, a speaker or a receiver. The speaker may be used for a general purpose, such as multimedia play or recording play. The receiver may be used to receive an incoming call. According to an embodiment, the receiver may be implemented separately from the speaker or may be implemented as a part of the speaker.

The display module 160 may visually provide information to the outside (e.g., the user) of the electronic device 101. The display module 160 may include, for example, a display, a hologram device, or a control circuit for controlling a projector and a corresponding device. According to an embodiment, the display module 160 may include a touch sensor configured to sense a touch, or a pressure sensor configured to measure the strength of force generated by the touch.

The audio module 170 may convert sound to an electrical signal, or reversely, may convert an electrical signal to sound. According to an embodiment, the audio module 170 may obtain sound through the input module 150, or may output sound through the sound output module 155, or through an external electronic device (e.g., the electronic device 102, a speaker, or a headphone) directly or wirelessly connected with the electronic device 101.

The sensor module 176 may sense an operation state (e.g., power or a temperature) of the electronic device 101 or an external environment state (e.g., a user state), and may generate an electrical signal or a data value corresponding the sensed state. According to an embodiment, the sensor module 176 may include, for example, a gesture sensor, a gyro sensor, a barometric pressure sensor, a magnetic sensor, an acceleration sensor, a grip sensor, a proximity sensor, a color sensor, an infrared (IR) sensor, a biometric sensor, a temperature sensor, a humidity sensor, or an illumination sensor.

The interface 177 may support one or more specified protocols that may be used to directly and wirelessly connect the electronic device 101 with an external electronic device (e.g., the electronic device 102). According to an embodiment, the interface 177 may include, for example, an HDMI (high-definition multimedia interface), a USB (universal serial bus) interface, an SD card interface, or an audio interface.

The connecting terminal 178 may include a connector that may allow the electronic device 101 to be physically connected with an external electronic device (e.g., the electronic device 102). According to an embodiment, the connecting terminal 178 may include, for example, a HDMI connector, an USB connector, an SD card connector, or an audio connector (e.g., a headphone connector).

The haptic module 179 may convert an electrical signal to a mechanical stimulation (e.g., vibration or movement) or an electrical stimulation which the user may perceive through the sense of touch or the sense of movement. According to an embodiment, the haptic module 179 may include, for example, a motor, a piezoelectric sensor, or an electrical stimulation device.

The camera module 180 may shoot a still image or a video image. According to an embodiment, the camera module 180 may include one or more lenses, image sensors, image signal processors, or flashes (or electrical flashes).

The power management module 188 may manage the power which is supplied to the electronic device 101. According to an embodiment, the power management module 188 may be implemented, for example, as at least part of a power management integrated circuit (PMIC).

The battery 189 may power at least one component of the electronic device 101. According to an embodiment, the battery 189 may include, for example, a primary cell not rechargeable, a secondary cell rechargeable, or a fuel cell.

The communication module 190 may establish a direct (or wired) communication channel or a wireless communication channel between the electronic device 101 and an external electronic device (e.g., the electronic device 102, the electronic device 104, or the server 108) and may perform communication through the established communication channel. The communication module 190 may include one or more communication processors which are operated independently of the processor 120 (e.g., an application processor) and support direct (or wired) communication or wireless communication. According to an embodiment, the communication module 190 may include a wireless communication module 192 (e.g., a cellular communication module, a short range wireless communication module, or a global navigation satellite system (GNSS) communication module) or a wired communication module 194 (e.g., a local area network (LAN) communication module or a power line communication module). The corresponding communication module among these communication modules may communicate with an external electronic device 104 through a first network 198 (e.g., a short-range communication network such as Bluetooth, wireless fidelity (WiFi) direct or infrared data association (IrDA)) or a second network 199 (e.g., long-range wireless communication network such as a legacy cellular network, 5G networks, next-generation communication networks, Internet, or computer networks (e.g., LAN or WAN)). The above-described kinds of communication modules may be integrated in one component (e.g., a single chip) or may be implemented with a plurality of components (e.g., a plurality of chips) which are independent of each other. The wireless communication module 192 may identify or authenticate the electronic device 101 within a communication network, such as the first network 198 or the second network 199, by using subscriber information (e.g., international mobile subscriber identity (IMSI)) stored in the subscriber identification module 196.

The wireless communication module 192 may support a 5G network and a next-generation communication technology after a 4G network, for example, a new radio (NR) access technology. The NR access technology may support enhanced mobile broadband (eMBB), massive machine type communications (mMTC), or ultra-reliable and low-latency communications (URLLC). For example, the wireless communication module 192 may support a high frequency band (e.g., mmWave band) to achieve a high data transfer rate. The wireless communication module 192 may support various technologies for securing performance in a high frequency band, for example, technologies such as beamforming, massive multiple-input and multiple-output (massive MIMO), full dimensional MIMO (FD-MIMO), an array antenna, analog beam-forming, and a large scale antenna. The wireless communication module 192 may support various requirements regulated in the electronic device 101, an external electronic device (e.g., the electronic device 104) or a network system (e.g., the second network 199). According to an embodiment, the wireless communication module 192 may support peak data rate (e.g., 20 Gbps or more) for eMBB implementation, loss coverage (e.g., 164 dB or less) for mMTC implementation, or U-plane latency (e.g., downlink (DL) of 0.5 ms or less and uplink (UL) of 0.5 ms or less, or round trip of 1 ms or less) for URLLC implementation.

The antenna module 197 may transmit a signal or a power to the outside (e.g., an external electronic device) or may receive a signal or a power from the outside. According to an embodiment, the antenna module 197 may include an antenna including a radiator formed of a conductor or a conductive pattern formed on a substrate (e.g., PCB). According to one embodiment, the antenna module 197 may include a plurality of antennas (e.g., an array antenna). In this case, at least one antenna suitable for a communication scheme used in a communication network such as the first network 198 or the second network 199 may be selected, for example, by the communication module 190 from the plurality of antennas. The signal or power may be exchanged between the communication module 190 and an external electronic device through the selected at least one antenna or may be received from the external electronic device through the selected at least one antenna and the communication module 190. According to some embodiments, other parts (e.g., radio frequency integrated circuit (RFIC)) may be additionally formed as a part of the antenna module 197 in addition to the radiator.

According to various embodiments, the antenna module 197 may form an mmWave antenna module. According to an embodiment, the mmWave antenna module may include a printed circuit board (PCB), a radio frequency integrated circuit (RFIC), and a plurality of antennas (e.g., an array antenna). The RFIC may be disposed on or adjacent to a first surface (e.g., a bottom surface) of the PCB and may support a specified high frequency band (e.g., mmWave band). The plurality of antennas may be disposed on or adjacent to a second surface (e.g., a top surface or a side surface) of the PCB and may transmit or receive a signal in the specified high frequency band.

At least some of the components may be connected to each other through a communication scheme (e.g., a bus, a general purpose input and output (GPIO), a serial peripheral interface (SPI), or a mobile industry processor interface (MIPI)) between peripheral devices and may exchange signals (e.g., commands or data) with each other.

According to an embodiment, the command or data may be transmitted or received between the electronic device 101 and the external electronic device 104 through the server 108 connected to the second network 199. Each of the external electronic device 102 or 104 may be a device of which the type is the same as or different from that of the electronic device 101. According to an embodiment, all or a part of operations to be executed by the electronic device 101 may be executed in one or more external electronic devices among the external electronic devices 102, 104, or 108. For example, when the electronic device 101 needs to perform any function or service automatically or in response to a request from the user or any other device, the electronic device 101 may additionally request one or more external electronic devices to perform at least part of the function or service, instead of internally executing the function or service. The one or more external electronic devices which receive the request may execute at least a part of the function or service thus requested or an additional function or service associated with the request, and may provide a result of the execution to the electronic device 101. The electronic device 101 may process the result as it is or additionally, and may provide a result of the processing as at least a part of the response to the request. To this end, for example, cloud computing, distributed computing, mobile edge computing (MEC), or client-server computing may be used. For example, the electronic device 101 may provide an ultra-low latency service by using distributed computing or mobile edge computing. In another embodiment, the external electronic device 104 may include an Internet of Things (IoT) device. The server 108 may be an intelligent server using machine learning and/or a neural network. According to an embodiment, the external electronic device 104 or the server 108 may be included in the second network 199. The electronic device 101 may be applied to an intelligent service (e.g., a smart home, a smart city, a smart car, or a healthcare) based on 5G communication technology and IoT-related technology.

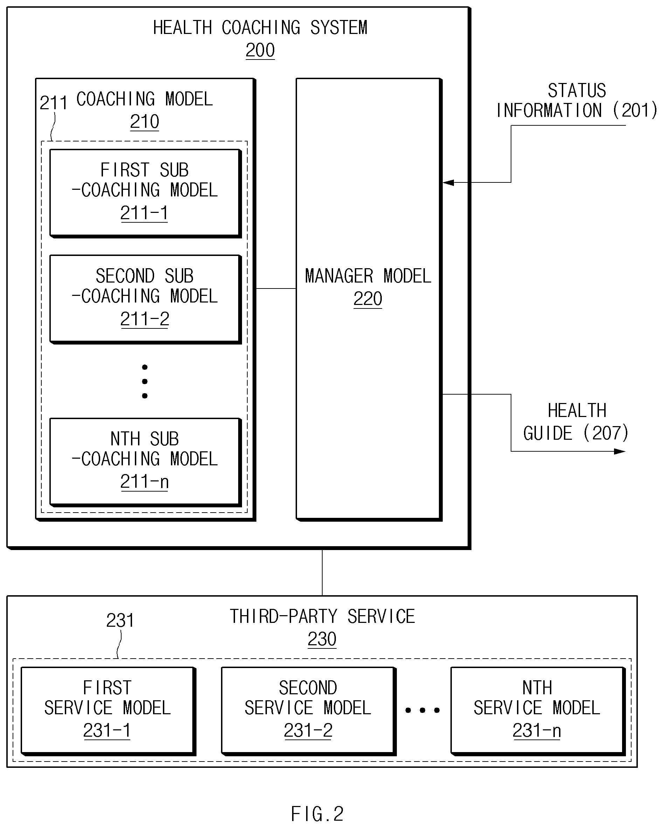

According to various embodiments, the electronic device 101 may provide an optimized health guide to a user by using coaching information obtained from a plurality of coaching models. This is described in detail below with reference to FIGS. 2 to 15. Moreover, at least one embodiment among various embodiments described with reference to FIGS. 2 to 15 may be combined with other embodiments.

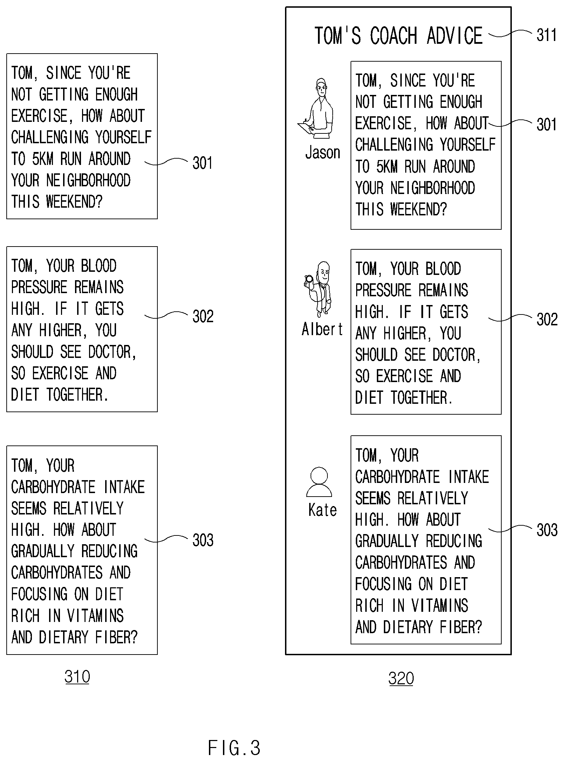

FIG. 2 is a diagram illustrating a health coaching system 200, according to various embodiments. FIG. 3 is a diagram illustrating a coaching operation of the health coaching system 200, according to various embodiments.

Referring to FIG. 2, the health coaching system 200 according to various embodiments may include a coaching model 210 configured to generate coaching information and a manager model 220 configured to manage the coaching model 210.

The aforementioned components of the health coaching system 200 are examples, and various embodiments are not limited thereto. For example, the health coaching system 200 may include more components than those illustrated in FIG. 2.

According to various embodiments, the manager model 220 may request the coaching model 210 to generate coaching information. The coaching information may represent a user's behavior and/or awareness required for the user's state (e.g., a health state, a food intake state, a sleep state, or a medication state) to reach a specified target value.

For example, the coaching information may indicate that there is a need for actions such as exercise and diet to reduce blood pressure. However, this is merely an example, and various embodiments are not limited thereto. For example, the coaching information may coach the electronic device 101 to reach a specified target state. Additionally or alternatively, like expert advice (e.g., information about diseases that could develop from a current condition or information related to medications or exercise that should be taken to improve (or maintain) health from the current condition) regarding the user's condition, various types of information capable of assisting the user in making decisions or solving problems to improve its health status may also be provided as the coaching information.

According to an embodiment, the manager model 220 may request the generation of coaching information based on status information 201 associated with the user (and/or the electronic device 101). The status information 201 may include at least some of information (e.g., posture information and/or biometric information) collected through the sensor module 176, user-related profile information (e.g., gender, hobbies, height, weight, or interests), user-related health information (e.g., disease, prescription medication, side effects, or medical history), and user-related activity information (e.g., exercise status, food intake status, or work status). However, these are merely examples, and various other pieces of information in addition to the aforementioned information may be used as the status information 201. Furthermore, the status information 201 may be acquired by the electronic device 101 or from an external electronic device (e.g., the electronic device 102, the electronic device 104, or the server 108).

For example, as part of an operation of requesting the generation of coaching information, the manager model 220 may generate a coaching request query based on the status information 201 and may provide the coaching request query to the coaching model 210. For example, the coaching request query may be generated in the form of a natural language sentence (e.g., "A 35-year-old man who mainly eats carbohydrate, currently undergoing two weeks of light exercise including an average of 30 minutes of walking per day, and has a blood pressure reading of 140 (systolic)/90 (diastolic)") or in the form of parameters (e.g., carbohydrates/male/35 years old/walking exercise).

According to various embodiments, the coaching model 210 may be configured to generate coaching information based on information (e.g., a coaching request query) input from the manager model 220. For example, the coaching model 210 may be composed of a plurality of sub-coaching models 211 (e.g., first to nth sub-coaching models 211-1 to 211-n). In this regard, each of the sub-coaching models 211 may generate coaching information by using information input from the manager model 220 as input data.

According to one embodiment, the sub-coaching models 211 may be configured to generate coaching information for different coaching fields (e.g., categories). For example, as illustrated in 310 of FIG. 3, the first sub-coaching model 211-1 may generate first coaching information 301 related to a first coaching field (e.g., fitness), the second sub-coaching model 211-2 may generate second coaching information 302 related to a second coaching field (e.g., chronic disease), and the nth sub-coaching model 211-n may generate third coaching information 303 related to a nth coaching field (e.g., nutrition).

For example, the first sub-coaching model 211-1 related to the first coaching field (e.g., fitness) may extract fitness-related features (e.g., age, gender, exercise time, and exercise type) from a coaching request query (e.g., "A 35-year-old man who mainly eats carbohydrate, currently undergoing two weeks of light exercise including an average of 30 minutes of walking per day, and has a blood pressure reading of 140 (systolic)/90 (diastolic)") and may generate the first coaching information 301 (e.g., recommending walking exercise in consideration of lack of exercise) based on this.

Similarly, the second sub-coaching model 211-2 related to the second coaching category (e.g., chronic disease) and the nth sub-coaching model 211-n related to the nth coaching category (e.g., nutrition) may also extract necessary features from the coaching request query, and may generate the second coaching information 302 (e.g., recommend exercise and diet related to blood pressure) and the third coaching information 303 (e.g., recommend diet improvement centered on vitamins and dietary fiber) based on this.

However, this is merely an example, and various embodiments are not limited thereto. For example, the sub-coaching model 211 may be configured to generate fine-tuned coaching information for a single learned coaching category. For example, the first sub-coaching model 211-1 may generate fourth coaching information (e.g., aerobic exercise information) related to the first coaching category (e.g., fitness), the second sub-coaching model 211-2 may generate fifth coaching information (e.g., anaerobic exercise information) related to the first coaching category (e.g., fitness), and the nth sub-coaching model 211-n may generate sixth coaching information (e.g., diet information) related to the first coaching category (e.g., fitness).

According to various embodiments, the manager model 220 may generate (e.g., output) a health guide 207 based on coaching information (e.g., the first coaching information 301 to the third coaching information 303) generated by the coaching model 210 (e.g., the sub-coaching model 211). The health guide 207 may induce changes in the user's behavior and/or awareness based on coaching information. For example, the coaching information may indicate the need for exercise and diet to reduce blood pressure, and the manager model 220 may generate the health guide 207 that instructs and/or encourages exercise and diet.

According to an embodiment, the health guide 207 may be a guide screen 311 composed of at least a portion of the first coaching information 301, the second coaching information 302, and the third coaching information 303 generated by the first sub-coaching model 211-1, the second sub-coaching model 211-2, and the nth sub-coaching model 211-n, as illustrated in 320 of FIG. 3. In this regard, in generating the health guide, the manager model 220 may prevent conflicts between the coaching information (e.g., the first coaching information 301, the second coaching information 302, and the third coaching information 303). For example, when the first coaching information 301 indicates recommending running for 5 km or more and the second coaching information 302 indicates recommending light exercise for blood pressure management, the manager model 220 may recommend light walking exercise rather than running for 5 km for blood pressure management.

Additionally or alternatively, the health coaching system 200 according to various embodiments may include a third-party service 230. The third-party service 230 may include a plurality of service models 231 (e.g., a first service model 231-1, a second service model 231-2, and an nth service model 231-n).

According to an embodiment, at least one of the plurality of service models 231 may be a sub-coaching model capable of being provided as a plug-in service. For example, the plurality of service models 231 may be created, updated, or removed periodically or intermittently by a service provider. In this regard, the manager model 220 may add at least one (e.g., the first service model 231-1) of the plurality of service models 231 to the coaching model 210. According to an embodiment, the service model 231 that is purchased by a user for a predetermined fee or free of charge may be added to the coaching model 210.

At least a portion of the health coaching system 200 described above may be part of the electronic device 100 described above with reference to FIG. 1. According to an embodiment, all components (e.g., the coaching model 210 and the manager model 220) included in the health coaching system 200 may be included within the electronic device 101 (e.g., the memory 130) or externally (e.g., the server 108). According to an embodiment, some components of the health coaching system 200 (e.g., the manager model 220) may be included within the electronic device 101, while other components (e.g., the coaching model 210) may be included externally to the electronic device 101.

As described above, the health coaching system 200 (e.g., the manager model 220) according to various embodiments may provide the meaningful health guide 207 that integrates various coaching fields based on coaching information generated by the plurality of sub-coaching models 211.

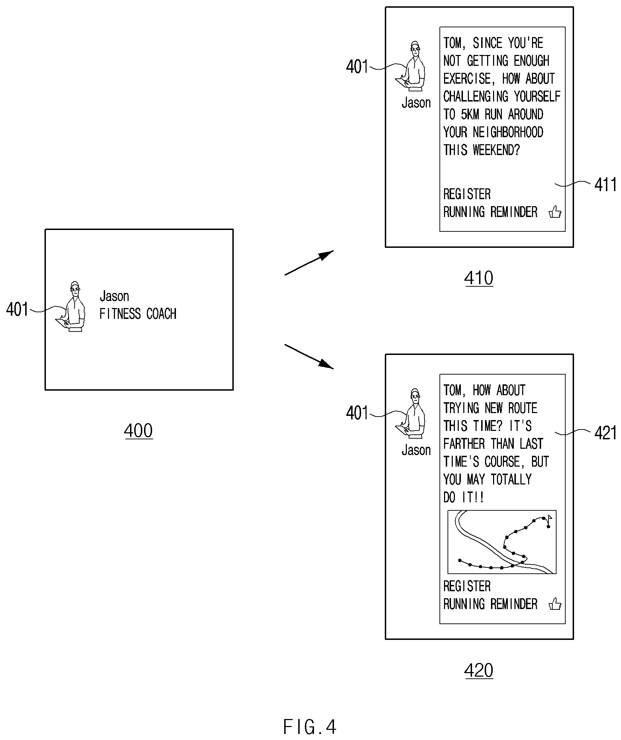

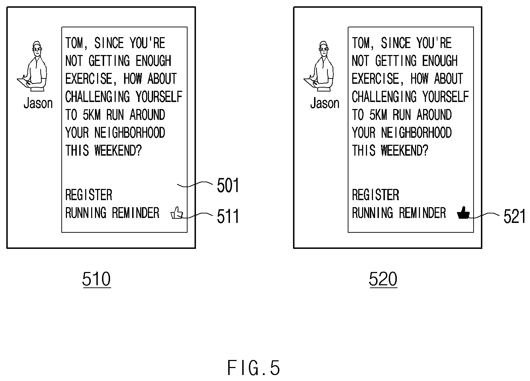

Additionally or alternatively, the health coaching system 200 (e.g., the manager model 220) according to various embodiments may additionally consider the user's preferences for the sub-coaching models 211 when generating the health guide 207. For example, the manager model 220 may generate the different health guides 207 based on the user's preferences for the sub-coaching models 211. This is described in detail below with reference to FIGS. 4 and 5.

FIG. 4 is a diagram illustrating the health guide 207, according to various embodiments. Moreover, FIG. 5 is a diagram illustrating a preference for a sub-coaching model 401, according to various embodiments.

Referring to FIG. 4, the manager model 220 according to various embodiments may generate different health guides 207 according to preferences for the sub-coaching model 401 (e.g., the sub-coaching models 211).

According to an embodiment, when a user is determined to have a relatively low preference for the sub-coaching model 401, the manager model 220 may generate a first health guide 411 including first-level information as a health guide for the corresponding sub-coaching model 401, as illustrated in 400 and 410 of FIG. 4. For example, the first health guide 411 may include first-level information indicating situations, where exercise is lacking, and recommending exercise accordingly.

According to an embodiment, when the user is determined to have a relatively high preference for the sub-coaching model 401, the manager model 220 may generate a second health guide 421, as illustrated in 400 and 420 of FIG. 4, for the sub-coaching model 401, that includes second-level information higher than the first-level information. The second health guide 421 may include more information or specific information than the first health guide 411. For example, the second health guide 421 may include the second-level information indicating situations where exercise is lacking, and suggesting exercise courses, instead of recommending exercise accordingly. Additionally or alternatively, the second health guide 421 may further include at least a portion of the coaching information provided by the other sub-coaching model 401. For example, the manager model 220 may provide the second health guide 421 by integrating at least a portion of the coaching information of the sub-coaching model 401 that the user most prefers with coaching information (e.g., at least a portion of the coaching information) provided by at least a portion of the coaching models that the user next prefers. Furthermore, the second health guide 421 may provide the second health guide 421 by integrating at least a portion of the coaching information of the sub-coaching model 401 that the user relatively prefers with coaching information (e.g., at least a portion of the coaching information) provided by at least a portion of the coaching models that the user relatively dislikes.

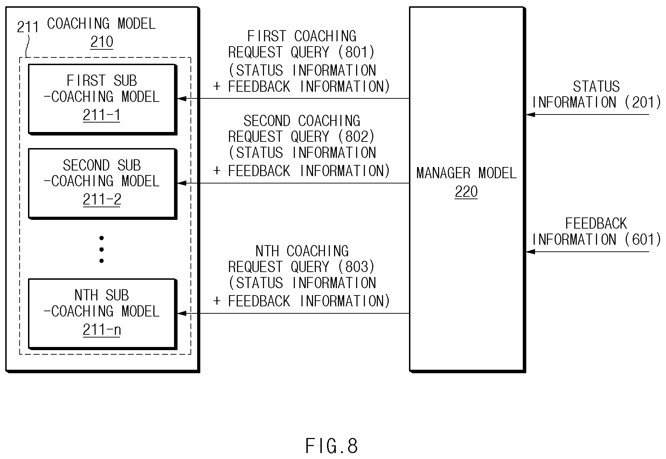

In this regard, the manager model 220 according to various embodiments may generate a coaching request query based on the status information 201, feedback information 601, and/or coaching information generated by the sub-coaching model 211, as described below with reference to FIGS. 8 to 11.

Additionally or alternatively, in determining the preference of the sub-coaching model 211, the manager model 220 according to various embodiments may consider user feedback on the coaching information generated by the sub-coaching model 211. The user feedback may include at least one of a positive evaluation, a negative evaluation, or a neutral evaluation of the coaching information.

According to an embodiment, the sub-coaching model 211 that outputs coaching information with the positive evaluation (alternatively, the negative evaluation or the neutral evaluation) may correspond to a sub-coaching model that satisfies a specified condition. For example, whenever coaching information is generated by each sub-coaching model 211, the manager model 220 may output item 511, which enables user feedback. The item 511, which enables the user feedback, may include at least one of an item enabling the positive evaluation, an item enabling the negative evaluation, or an item enabling the neutral evaluation. For example, the item 511 may be output together with coaching information 501 (e.g., the first coaching information 301), as illustrated in 510 of FIG. 5. Accordingly, the manager model 220 may detect a user input selecting (521) the item 511, as illustrated in 520 of FIG. 5, and may manage (e.g., store) user feedback.

Additionally or alternatively, the user feedback may include whether the coaching information was successfully completed. In an embodiment, when the user performs the coaching information (e.g., running 5 km) output by the sub-coaching model 211, the feedback on the sub-coaching model 211 (or the coaching information) may be considered as the positive feedback. Furthermore, when the user fails to achieve the coaching information through actions, the feedback on the sub-coaching model 211 (or the coaching information) may be considered as negative or neutral feedback.

As described above, the manager model 220 according to various embodiments may provide the health guide 207 based on the coaching information generated by the plurality of sub-coaching models 211.

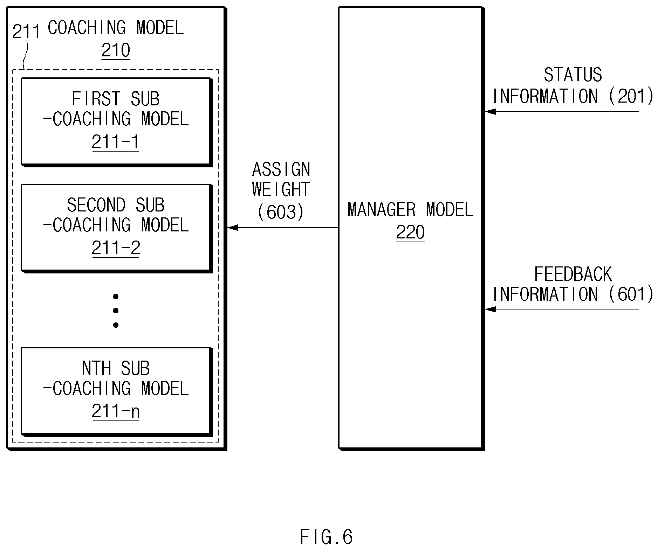

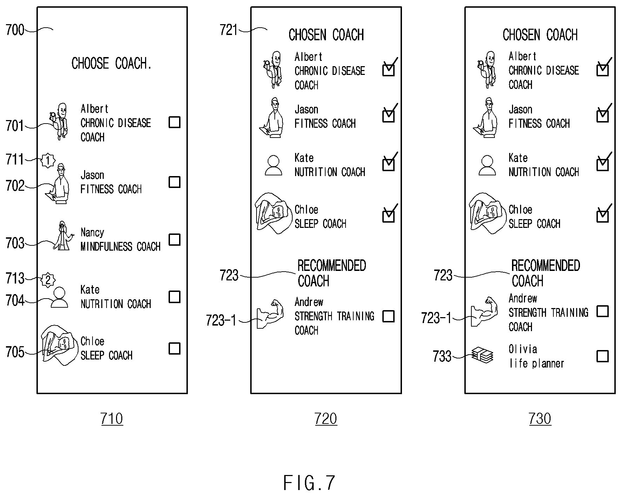

Additionally or alternatively, the manager model 220 according to various embodiments may assign a weight to coaching information generated by at least some (e.g., the first sub-coaching model 211-1) of the plurality of sub-coaching models 211 and then may use the weighted coaching information to generate the health guide 207. This is described in detail below with reference to FIGS. 6 and 7.

FIG. 6 is a diagram illustrating an operation of the manager model 220 assigning a weight to the sub-coaching model 211, according to various embodiments. Moreover, FIG. 7 is a diagram illustrating an operation of assigning a weight to the sub-coaching model 211, according to various embodiments.

Referring to FIG. 6, the manager model 220 according to various embodiments may assign weights to at least some of the plurality of sub-coaching models 211 (e.g., the first to nth sub-coaching models 211-1 to 211-n). Assigning weights to the sub-coaching models 211, as described below, may be interpreted as requesting higher-level coaching information.

According to various embodiments, in selecting the weighting targets, the manager model 220 may consider the user's preferences for the sub-coaching models 211. As previously mentioned, the preferences may be associated with user feedback on the coaching information generated by each sub-coaching model 211.

In this regard, the manager model 220 may select a target, to which a weight is assigned (hereinafter referred to as a "weight-assignment target"), from the plurality of sub-coaching models 211 based on the feedback information 601. For example, among the plurality of sub-coaching models 211, only a sub-coaching model that has a history of generating coaching information receiving positive feedback from the user and/or a sub-coaching model selected by the user may be selected as the target, to which a weight is assigned.

For example, a situation where positive feedback has been detected for the first sub-coaching model 211-1, and negative or neutral feedback has been detected for the remaining sub-coaching models (e.g., the second to nth sub-coaching model 211-2 to 211-n) from among the first to nth sub-coaching models 211-1 to 211-n may be assumed. In this situation, when the health coaching system 200 is executed, the manager model 220 may assign a weight 603 to the first sub-coaching model 211-1. For this reason, coaching information of a first level may be obtained through the unweighted second to nth sub-coaching model 211-2 to 211-n, and coaching information of a second level higher than the first-level may be obtained through the weighted first sub-coaching model 211-1.

Additionally or alternatively, the manager model 220 may deactivate the unweighted sub-coaching model and may activate only the weighted sub-coaching model.

For example, a situation where positive feedback has been detected for the first sub-coaching model 211-1, and negative or neutral feedback has been detected for the remaining sub-coaching models (e.g., the second to nth sub-coaching model 211-2 to 211-n) from among the first to nth sub-coaching models 211-1 to 211-n may be assumed. In this situation, when the health coaching system 200 is executed, the manager model 220 may allow only the first sub-coaching model 211-1 to be activated.

According to various embodiments, in selecting the weight-assignment target, the manager model 220 may consider the status information 201 related to a user (and/or the electronic device 101).

According to an embodiment, the manager model 220 may select the sub-coaching model 211 appropriate for a user based on the status information 201 as the weight-assignment target. For example, the manager model 220 may determine the user's status based on the status information 201 and may assign a weight to the sub-coaching model 211 corresponding to the user's status. The user's status may include at least one of a vehicle movement situation, a walking movement situation, a rest situation, or a work situation.

For example, when the vehicle movement situation is determined, the manager model 220 may exclude the first sub-coaching model 211 related to the first coaching field (e.g., fitness) from the weight-assignment target, as exercise is not appropriate while a vehicle is moving. Additionally, when a walking movement situation is determined, the manager model 220 may first select the sub-coaching model 211 related to the first coaching field (e.g., fitness) as the weight-assignment target, as exercise is possible while the user is walking.

Additionally or alternatively, the manager model 220 according to various embodiments may select the weight-assignment target in consideration of a combination of the status information 201 related to the user (and/or the electronic device 101) and the user's preference for the sub-coaching model 211 described above.

According to various embodiments, in selecting the weight-assignment target, the manager model 220 may select the coaching model 211 selected by an input (e.g., a voice input, a touch input, or a gesture input) as the weight-assignment target. According to an embodiment, the manager model 220 may output a list of a plurality of sub-coaching models and may select the weight-assignment target based on an input (e.g., a touch input, a voice input, or a gesture input) to the list.

For example, as illustrated in 710 of FIG. 7, when the coaching model 210 is composed of five sub-coaching models 211 (e.g., a chronic disease coach 701, a fitness coach 702, a mindfulness coach 703, a nutrition coach 704, and a sleep coach 705), the manager model 220 may output a list 700 including identification information (e.g., a name or an image of the sub-coaching model) for each sub-coaching model and check boxes for selecting or deselecting each sub-coaching model.

According to an embodiment, the manager model 220 according to various embodiments may select the weight-assignment target in consideration of a combination of a user's input for selecting the coaching model 211 and the status information 201 associated with the aforementioned user (and/or the electronic device 101).

Additionally or alternatively, in outputting the list 700 for the coaching model 210, the manager model 220 may assign indicators 711 and 713 indicating a preference for at least one of the sub-coaching models 211. For example, in the illustrated drawing, the first indicator 711 indicating the highest preference for the fitness coach 702 and the second indicator 713 indicating the next highest preference for the nutrition coach 704 may be identified. The indicators 711 and 713 may allow the user to easily distinguish between relatively preferred and relatively non-preferred targets among the sub-coaching models 211.

Additionally or alternatively, the manager model 220 according to various embodiments may recommend assigning an additional weight for at least one other sub-coaching model 721 associated with the sub-coaching model 721 selected as the weight-assignment target, as illustrated in 720 of FIG. 7.

In an embodiment, the manager model 220 may recommend, along with the sub-coaching model 721 selected as the weight-assignment target, another sub-coaching model 723 capable of helping the user's condition (e.g., health condition) reach the specified target.

For example, the manager model 220 may select the user's most preferred target (e.g., the fitness coach 702) among the selected sub-coaching model 721 (e.g., the chronic disease coach 701, the fitness coach 702, the nutrition coach 704, and the sleep coach 705) and may recommend a coach related to the selected target (e.g., a strength training coach 723-1 trained with specialized fitness information, knowledge, and data).

Additionally or alternatively, as illustrated in 730 of FIG. 7, the manager model 220 according to various embodiments may provide a list 733 of another sub-coaching model (e.g., Olivia (life planner)) that may be additionally selected by the user, along with the sub-coaching model 721 selected as the weight-assignment target and another recommended sub-coaching model 723. For example, the manager model 220 may also provide information about at least one sub-coaching model that is currently selected or is not recommended.

As described above, the manager model 220 according to various embodiments may utilize coaching information generated by the plurality of sub-coaching models 210 to generate the health guide 207. For example, the manager model 220 may request the generation of coaching information by providing a coaching request query to the coaching model 210. This will be described in detail with reference to FIGS. 8 to 10 below.

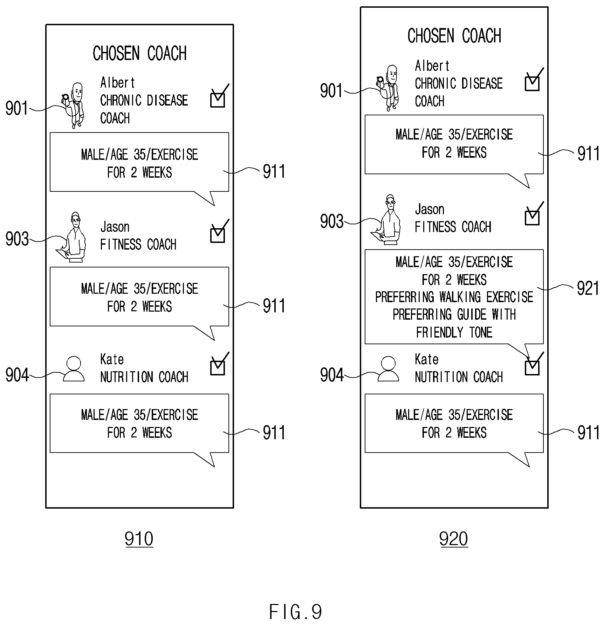

FIG. 8 is a diagram illustrating an operation of the manager model 220 providing a coaching request query, according to various embodiments. Moreover, FIG. 9 is a diagram illustrating a coaching query request, according to various embodiments.

Referring to FIG. 8, as part of an operation of requesting the generation of coaching information, the manager model 220 according to various embodiments may generate a coaching request query based on the status information 201 and/or the feedback information 601 and may provide the coaching request query to the coaching model 210. For example, the manager model 220 may provide a first coaching request query 801 to the first sub-coaching model 211-1, may provide a second coaching request query 802 to the second sub-coaching model 211-2, and may provide an nth coaching request query 803 to the nth sub-coaching model 211-n.

According to an embodiment, the manager model 220 may generate a single coaching request query based on the status information 201 and may provide the coaching request query to each of the sub-coaching models 211. For example, as illustrated in 910 of FIG. 9, the manager model 220 may provide substantially the same coaching request query 911 (e.g., male/age 35/exercise for 2 weeks) to a first sub-coaching model 901 (e.g., the first sub-coaching model 211-1), a second sub-coaching model 903 (e.g., the second sub-coaching model 211-2), and a third sub-coaching model 904 (e.g., the nth sub-coaching model 211-n). According to an embodiment, the coaching request query provided to at least one sub-coaching model may be output through a screen. For example, the coaching request query may be output in a conversational manner, for example, through a speech bubble.

Additionally or alternatively, in generating a coaching request query, the manager model 220 may consider the user's preference for the sub-coaching models 211. As previously mentioned, the preferences may be associated with each of the sub-coaching models 211 and/or user feedback on coaching information generated by each of the sub-coaching models 211.

According to one embodiment, on the basis of the preferences for the sub-coaching models 211, the manager model 220 may generate a coaching request query including status information of a first level, or may generate a coaching request query including status information of a second level higher than status information of the first level. The status information of the second level may include more information or more specific information than the status information of the first level.

For example, among the sub-coaching models 211, the second sub-coaching model 903 may be determined to have the preference satisfying the specified condition, while the remaining sub-coaching models (e.g., the first sub-coaching model 901 and the third sub-coaching model 904) may be determined to have preferences that do not satisfy the specified condition.

In this case, as illustrated in 920 of FIG. 9, the manager model 220 may generate the coaching request query 911 (e.g., male/age 35/exercise completed for 2 weeks) including status information of the first level for the first sub-coaching model 901 and the third sub-coaching model 904 that do not satisfy the specified condition.

Additionally, the manager model 220 may generate a coaching request query 921 including the status information of the second level (e.g., male/35 years old/exercise for 2 weeks/preferring walking exercise, or preferring a guide with a friendly tone) for the second sub-coaching model 903 that has preferences that satisfy the specified condition.

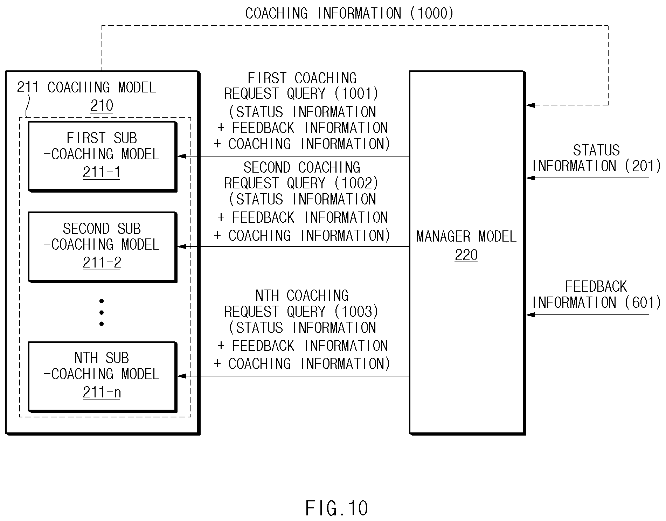

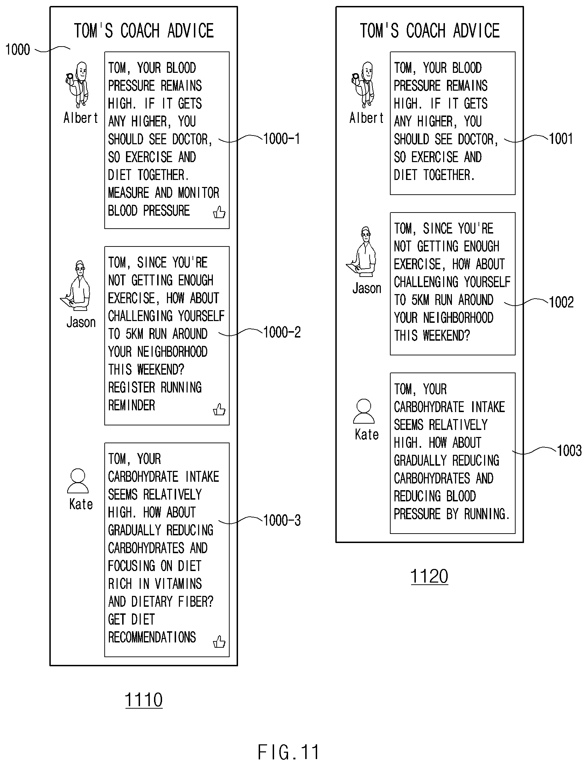

FIG. 10 is a diagram illustrating an operation of the manager model 220 providing a coaching request query, according to various embodiments. FIG. 11 is a diagram illustrating a coaching query request, according to various embodiments.

Referring to FIG. 10, as part of an operation of requesting the generation of coaching information, the manager model 220 according to various embodiments may generate a coaching request query and may provide the coaching request query to the coaching model 210. According to an embodiment, as described above with reference to FIG. 8, the manager model 220 may generate a coaching request query based on the status information 201 and/or the feedback information 601, and may provide the coaching request query to each of the sub-coaching models 211.

Additionally or alternatively, in generating the coaching request query, the manager model 220 according to various embodiments may additionally utilize coaching information 1000 generated by the sub-coaching model 210. According to an embodiment, the coaching information output by the sub-coaching model may be used as a coaching request query input to another sub-coaching model.

For example, 1110 of FIG. 11 represents the coaching information 1000 generated by the sub-coaching model 210. For example, the first to nth sub-coaching models 211-1 to 211-n may be interpreted as generating first to third coaching information 1000-1 to 1000-3. Referring to this, as illustrated in 1120 of FIG. 11, first coaching request query 1001 provided to the first sub-coaching model 211-1 may be generated by a combination of the status information 201, the feedback information 601, and second coaching information 1002 (e.g., running exercise recommendation) (and/or third coaching information 1003 (e.g., diet control recommendation)) generated by the second sub-coaching model 211-2 (and/or the nth sub-coaching model 211-n). In addition, the second coaching request query 1002 provided to the second sub-coaching model 211-2 may be generated by a combination of the status information 201, the feedback information 601, and the first coaching information 1001 (and/or third coaching information 1003) generated by the first sub-coaching model 211-1 (e.g., blood pressure reduction recommendation) (and/or the nth sub-coaching model 211-n (e.g., diet control recommendation)). The third coaching request query 1003 provided to the nth sub-coaching model 211-n may be generated by a combination of the status information 201, the feedback information 601, and the first coaching information 1001 (e.g., blood pressure reduction recommendation) (and/or the second coaching information 1002 (e.g., running exercise recommendation)) generated by the first sub-coaching model 211-1 (and/or the second sub-coaching model 211-2.

However, this is merely an example, and various embodiments are not limited thereto. For example, the coaching information 1000 may only apply to a coaching request query provided to a highly preferred sub-coaching model (e.g., the second sub-coaching model 211-2).

For example, the first sub-coaching model 211-1 and the nth sub-coaching model 211-n, which have relatively low preferences, may be provided with a coaching request query 1111 generated based on the status information 201 and/or the feedback information 601. Furthermore, the second sub-coaching model 211-2, which has relatively high preferences, may additionally be provided with coaching information generated by the first sub-coaching model 211-1 and/or the nth sub-coaching model 211-n, in addition to the status information 201 and the feedback information 601.

As another example, coaching information generated by a sub-coaching model with a relatively high preference (e.g., the second sub-coaching model 211-2) may be included in queries provided to the first sub-coaching model 211-1 and the nth sub-coaching model 211-n, which have relatively low preferences. A coaching request query including the user's preferred information may be input to the first and nth sub-coaching model 211-1 and 211-n, which have relatively low preferences. As a result, the preference for the first sub-coaching model 211-1 and the nth sub-coaching model 211-n, which currently have relatively low preferences, may increase over time.

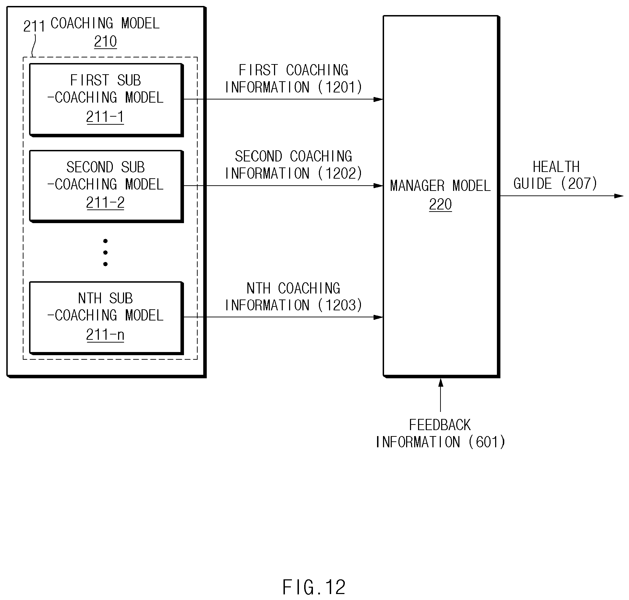

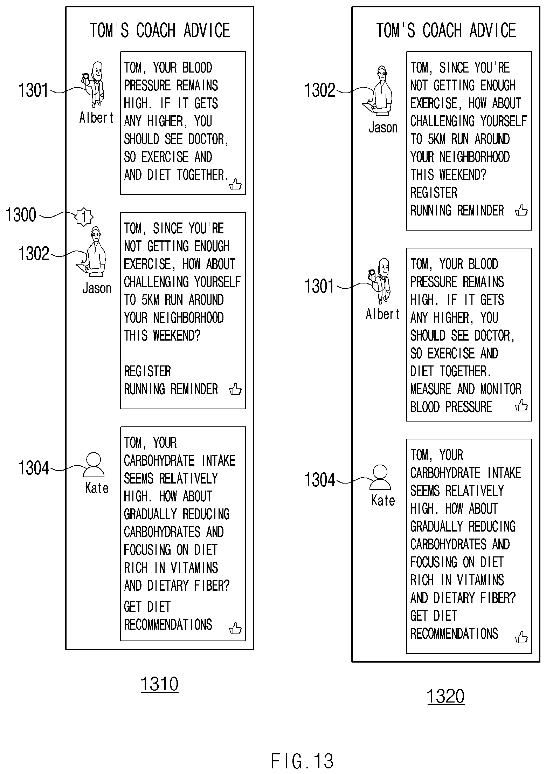

FIG. 12 is a diagram illustrating an operation of generating the health guide 207 of the manager model 220, according to various embodiments. Moreover, FIG. 13 is a drawing for describing a method of outputting the health guide 207 of the manager model 220, according to various embodiments.

Referring to FIG. 12, the manager model 220 according to various embodiments may provide a meaningful health guide 207 that combines various coaching fields based on coaching information (e.g., first to nth coaching information 1201 to 1203) generated by the plurality of sub-coaching models 211. Additionally or alternatively, as described above, in generating the health guide 207, the manager model 220 may additionally consider the user's preference for the sub-coaching model 211.

According to various embodiments, as illustrated in FIG. 13, the manager model 220 may provide a guide screen (e.g., the guide screen 311 of FIG. 3) composed of at least a portion of the coaching information (e.g., first coaching information 1301, second coaching information 1302, and third coaching information 1304) generated from the sub-coaching model 211.

In this regard, the manager model 220 according to various embodiments may output coaching information (e.g., the second coaching information 1302) provided from a sub-coaching model (e.g., the second sub-coaching model 211-2) that satisfies a specified condition, and coaching information (e.g., the first coaching information 1301 and the third coaching information 1304) provided from a coaching model (e.g., the first sub-coaching model 211-1 and the third sub-coaching model 211-3) that does not satisfy the specified condition in different ways.

According to one embodiment, as illustrated in 1310 of FIG. 13, the manager model 220 may assign an indicator 1300 indicating a preference to coaching information (e.g., the second coaching information 1302) provided from a sub-coaching model (e.g., the second sub-coaching model 211-2) that satisfies the specified condition from among the coaching information included in the guide screen 311. The indicator 1300 may enable a user to easily distinguish between relatively preferred coaching information and relatively non-preferred coaching information.

According to another embodiment, the manager model 220 may also change the output location of coaching information provided from a sub-coaching model, which satisfies the specified condition, from among the coaching information included in the guide screen 311. As shown in 1320 of FIG. 13, coaching information (e.g., the second coaching information 1302) provided from a sub-coaching model (e.g., the second sub-coaching model 211-2) that satisfies the specified conditions is output in priority order, and subsequently, coaching information (e.g., the first coaching information 1301 and the third coaching information 1304) provided from a sub-coaching model (e.g., the first sub-coaching model 211-1 and the third sub-coaching model 211-3) that does not satisfy the specified condition may be output.

According to another embodiment, the manager model 220 may differently output the amount of coaching information (e.g., the second coaching information 1302) provided from a sub-coaching model (e.g., the second sub-coaching model 211-2) that satisfies the specified condition, and the amount of coaching information (e.g., the first coaching information 1301 and the third coaching information 1304) provided from a sub-coaching model (e.g., the first sub-coaching model 211-1 and the third sub-coaching model 211-3) that does not satisfy a specified condition. For example, the coaching information (e.g., the second coaching information 1302) provided from a sub-coaching model (e.g., the second sub-coaching model 211-2) that satisfies the specified condition may be totally output. The coaching information (e.g., the first coaching information 1301 and the third coaching information 1304) provided from a sub-coaching model (e.g., the first sub-coaching model 211-1 and the third sub-coaching model 211-3) may only be partially output.

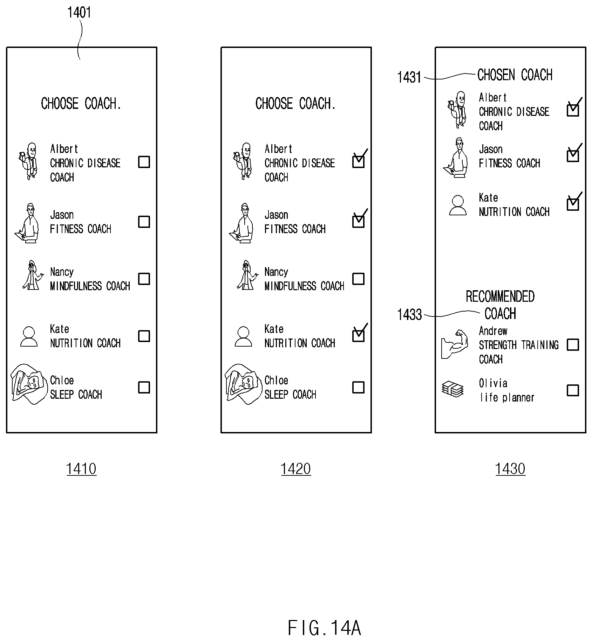

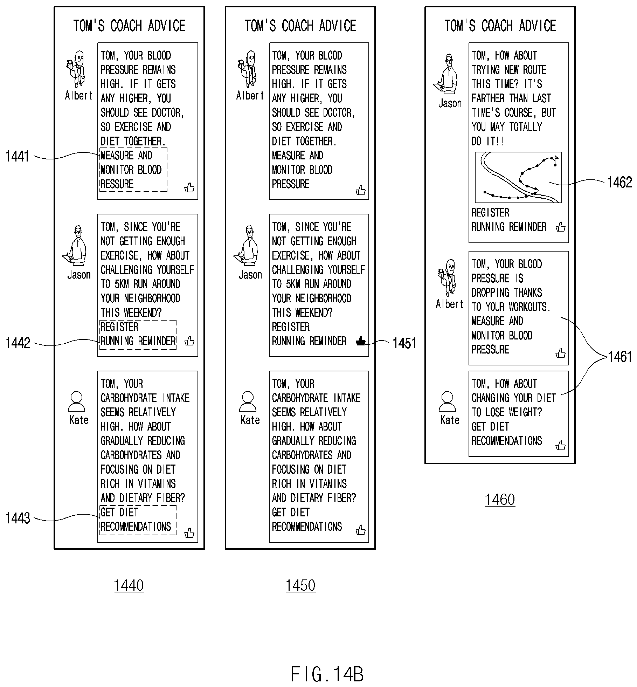

FIGS. 14A and 14B are diagrams illustrating sequential operations of the health coaching system 200, according to various embodiments. Moreover,

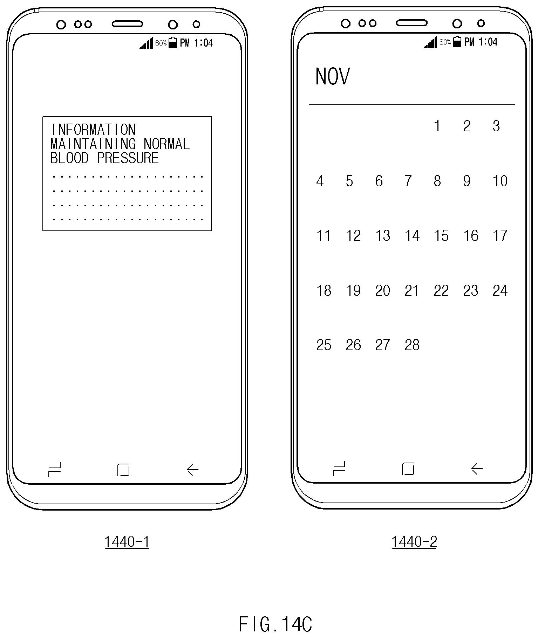

FIG. 14C is a diagram illustrating a health guide, according to various embodiments.

Referring to FIGS. 14A and 14B, in operation 1410, the health coaching system 200 (e.g., the manager model 220) according to various embodiments may output a list 1401 of the plurality of sub-coaching models 211. The plurality of sub-coaching models 211 may be sub-coaching models included in the coaching model 210. According to an embodiment, the health coaching system 200 (e.g., the manager model 220) may output the list 1401 in response to the execution of the health coaching system 200.

According to various embodiments, in operation 1420, the health coaching system 200 (e.g., the manager model 220) may select a preferred target from the plurality of sub-coaching models 211 based on an input (e.g., a voice input, a touch input, or a gesture input) for the list 1401. In this regard, the health coaching system 200 (e.g., the manager model 220) may provide a coaching request query to the sub-coaching model selected as the preferred target.

Additionally or alternatively, in operation 1430, the health coaching system 200 (e.g., the manager model 220) according to various embodiments may recommend additional selections for at least one other sub-coaching model 1433 associated with the sub-coaching model selected as the preferred target. In this regard, when an input is detected to add the other recommended sub-coaching model 1433, the health coaching system 200 (e.g., the manager model 220) may also provide a coaching request query for the other recommended sub-coaching model 1433.

In operation 1440, the health coaching system 200 (e.g., the manager model 220) according to various embodiments may output a guide screen (e.g., the guide screen 311 of FIG. 3) including at least a portion of the coaching information generated from a preferred sub-coaching model. For example, coaching information generated by preferred sub-coaching models may be provided to a single screen through a guide screen. Moreover, only coaching information (e.g., priority information), which satisfies a specified condition, from among coaching information generated by the preferred sub-coaching models may be provided through the guide screen. Furthermore, not only coaching information generated by the preferred sub-coaching models but also coaching information generated by another sub-coaching model not selected as the preferred sub-coaching model may be provided through the guide screen

According to an embodiment, the coaching information included in the guide screen may include additional information 1441, 1442, and 1443 (e.g., detailed information or related information) for each coaching information.

The additional information may be information capable of additionally linked to other applications or other electronic devices in relation to respective coaching information. For example, the additional information may be link information that links more specialized information related to coaching information. Furthermore, the additional information may be link information that links other applications or electronic devices that register coaching information.

For example, as illustrated in 1440-1 of FIG. 14C, when a user input for the additional information occurs, the health coaching system 200 (e.g., the manager model 220) may execute a specified application (e.g., a web browser) to output more specialized content (e.g., (e.g., related paper data) related to the coaching information (e.g., blood pressure management) provided from the sub-coaching model.

For example, as illustrated in 1440-2 of FIG. 14C, when a user input for the additional information occurs, the health coaching system 200 (e.g., the manager model 220) may execute a specified application (e.g., a schedule management application) to register coaching information (e.g., exercise management) provided from the sub-coaching model.

In operation 1450, the health coaching system 200 (e.g., the manager model 220) according to various embodiments may identify user feedback 1451 on coaching information generated from a preferred sub-coaching model. According to an embodiment, the health coaching system 200 (e.g., the manager model 220) may identify coaching information, for which a positive evaluation has been detected, from among coaching information.

In operation 1460, the health coaching system 200 (e.g., the manager model 220) according to various embodiments may update coaching information for which a positive evaluation has been detected. According to an embodiment, the health coaching system 200 (e.g., the manager model 220) may provide an additional coaching request query to the sub-coaching model that generated the coaching information for which a positive evaluation has been detected. For example, the additional coaching request query may include at least a portion of coaching information (e.g., coaching information generated from another sub-coaching model) 1461 for which a positive evaluation is not detected. In this regard, the health coaching system 200 (e.g., the manager model 220) may generate additional coaching information 1462 based on the additional coaching request query and may update the coaching information based on the additional coaching information 1462.

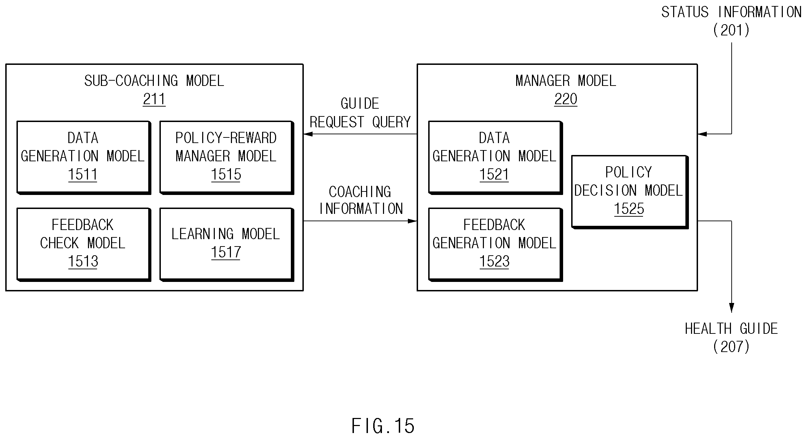

FIG. 15 is a diagram illustrating a configuration of the sub-coaching model 211 and the manager model 220, according to various embodiments.

Referring to FIG. 15, the manager model 220 according to various embodiments may include a data generation model 1521, a feedback generation model 1523, and a policy decision model 1525. According to one embodiment, the manager model 220 may include a generative model configured to generate new output data based on input data. However, this is merely an example, and various embodiments are not limited thereto. For example, the manager model 220 may be composed of various types of models other than the generative model.

According to an embodiment, the data generation model 1521 may generate a coaching request query based on the status information 201. According to an embodiment, the data generation model 1521 may generate a coaching request query corresponding to the sub-coaching model 211. Additionally or alternatively, the data generation model 1521 may consider feedback information in addition to the status information 201 to generate the coaching request query.

According to an embodiment, the data generation model 1521 may generate the health guide 207 based on the coaching information generated by the sub-coaching model 211. In an embodiment, the data generation model 1521 may generate the health guide 207 including at least a portion of the coaching information generated by each of the sub-coaching models 211. Additionally or alternatively, in generating the health guide 207, the data generation model 1521 may consider feedback information.

In an embodiment, the feedback generation model 1523 may identify user feedback on the coaching information generated by the sub-coaching model 211. The user feedback may include at least one of a positive evaluation, a negative evaluation, or a neutral evaluation of the coaching information. For example, the feedback generation model 1523 may evaluate the sub-coaching model 211 based on user feedback.

According to an embodiment, the policy decision model 1525 may determine a policy for the manager model 220. Furthermore, the policy decision model 1525 may perform fine-tuning on the sub-coaching model 211 based on the user feedback. For example, the policy decision model 1525 may re-train the sub-coaching model 211 by using a coaching request query generated based on the status information 201 and feedback information as a new data set.

According to various embodiments, the sub-coaching model 211 may include a data generation model 1511, a feedback check model 1513, a policy-reward manager model 1515, and a learning model 1517. In one embodiment, the sub-coaching model 211 may be a coaching model learned based on knowledge. However, this is merely an example, and various embodiments are not limited thereto.

In an embodiment, the data generation model 1511 may extract features necessary for generating coaching information from a coaching request query provided by the manager model 220. These extracted features may be used as input data for the learning model 1517.

In an embodiment, the feedback check model 1513 may periodically or aperiodically update preferences for the sub-coaching model 211.

In an embodiment, the policy-reward manager model 1515 may perform fine-tuning on the sub-coaching model 211. For example, the policy-reward manager model 1515 may retrain the learning model 1517 based on preferences.

In an embodiment, the policy-reward manager model 1515 may perform learning so as to determine a reward based on user feedback as the maximum value such that optimal coaching information is generated.

According to various embodiments, the electronic device 101 may include the display 160, the memory 130 that stores the health coaching system 200 including the first sub-coaching model 211-1, the second sub-coaching model 211-2 and the manager model 220 and the processor 120 electrically connected with the display 160 and the memory 130 and that is configured to display a health guide through the display 160 by controlling the health coaching system 200.

According to various embodiments, under the control of the processor 120, the manager model 220 may be configured to provide a first coaching request query based on the status information 201 related to a user, to the first sub-coaching model 211-1 and the second coaching sub model 211-2, to obtain the first coaching information 301 of a first level corresponding to the first coaching request query from the first sub-coaching model 211-1, to obtain the second coaching information 302 of a first level corresponding to the first coaching request query from the second sub-coaching model 211-2, to display the first health guide 311 including the first coaching information 301 and the second coaching information 302 through the display 160, based on a feedback about the first health guide 311, to select at least one of the first sub-coaching model 211-1 and the second sub-coaching model 211-2 as a preferred coaching model, when providing a second coaching request query based on the status information 201 related to the user to the first sub-coaching model 211-1 and the second sub-coaching model 211-2, to obtain the third coaching information 421 of a second level higher than the first level from the preferred coaching model among the first sub-coaching model 211-1 and the second sub-coaching model 211-2, and to obtain the fourth coaching information 411 of the first level from another sub-coaching model except for the preferred coaching model.

According to various embodiments, the manager model 220 may be configured to display a second health guide that at least a portion of the first health guide 311 is changed, through the display 160 based on the third coaching information and the fourth coaching information corresponding to the second coaching request query.

According to various embodiments, the manager model 220 may be configured to display the first item 511 related to feedback for the first coaching information and a second item related to feedback for the second coaching information together with the first health guide 311, and to determine the feedback about the first health guide 311 based on an input 521 for at least one of the first item 511 and the second item.

According to various embodiments, in providing the first coaching request query to the first sub-coaching model 211-1 and the second sub-coaching model 211-2, the manager model 220 may be configured to determine preferences for the first sub-coaching model 211-1 and the second sub-coaching model 211-2, to provide the first coaching request query based on first information corresponding to a first range among the status information 201 related to the user, to a model having a preference less than a specified threshold value among the first sub-coaching model 211-1 and the second sub-coaching model 211-2, and to provide the first coaching request query based on second information corresponding to a second range greater than the first range among the status information 201 related to the user, to a model having a preference larger than the specified threshold value among the first sub-coaching model 211-1 and the second sub-coaching model 211-2.

According to various embodiments, in providing the second coaching request query to the first sub-coaching model 211-1 and the second sub-coaching model 211-2, the manager model 220 may be configured to determine preferences for the first sub-coaching model 211-1 and the second sub-coaching model 211-2, and to provide the second coaching request query based on coaching information, which is obtained from a model having a preference larger than or equal to a specified threshold value among the first sub-coaching model 211-1 and the second sub-coaching model 211-2, to a model having a preference less than a specified threshold value among the first sub-coaching model 211-1 and the second sub-coaching model 211-2.

According to various embodiments, in providing the second coaching request query to the first sub-coaching model 211-1 and the second sub-coaching model 211-2, the manager model 220 may be configured to determine preferences for the first sub-coaching model 211-1 and the second sub-coaching model 211-2, and to provide the second coaching request query based on coaching information, which is obtained from a model having a preference less than a specified threshold value among the first sub-coaching model 211-1 and the second sub-coaching model 211-2, to a model having a preference larger than or equal to a specified threshold value among the first sub-coaching model 211-1 and the second sub-coaching model 211-2.

According to various embodiments, in providing the first coaching request query to the first sub-coaching model 211-1 and the second sub-coaching model 211-2, the manager model 220 may be configured to display the first coaching request query provided to the first sub-coaching model 211-1 and the second sub-coaching model 211-2 through the display 160.

According to various embodiments, in providing the second coaching request query to the first sub-coaching model 211-1 and the second sub-coaching model 211-2, the manager model 220 may be configured to display the second coaching request query provided to the first sub-coaching model 211-1 and the second sub-coaching model 211-2 through the display 160.

According to various embodiments, the manager model 220 may be configured to perform a fine-tuning to the first sub-coaching model 211-1 and the second sub-coaching model 211-2 based on feedback on the first health guide 311.

According to various embodiments, the manager model 220 may be configured to obtain an additional sub-coaching model from an external device based on feedback on the first health guide 311.

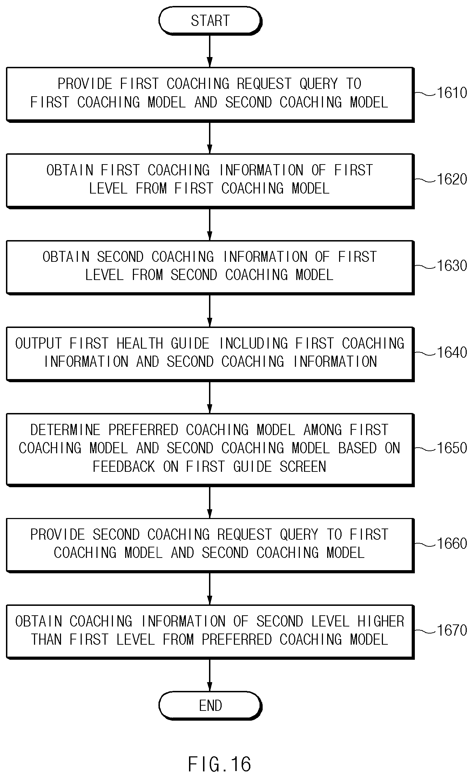

FIG. 16 is a flowchart illustrating the operation of the health coaching system 200, according to various embodiments. Moreover, each operation in the following embodiments may be sequentially performed, but is not necessarily sequentially performed. For example, the order of operations may be changed, and at least two operations may be performed in parallel. Also, at least one of the above-described operations may be omitted according to an embodiment.

Referring to FIG. 16, in operation 1610, the health coaching system 200 (e.g., the manager model 220) according to various embodiments may provide a first coaching request query to the first sub-coaching model and the second sub-coaching model. According to an embodiment, the health coaching system 200 (e.g., the manager model 220) may generate a coaching request query based on the status information 201 associated with a user (and/or the electronic device 101).

According to various embodiments, in operation 1620, the health coaching system 200 (e.g., the manager model 220) may obtain first coaching information of a first level from a first sub-coaching model.

According to various embodiments, in operation 1630, the health coaching system 200 (e.g., the manager model 220) may obtain second coaching information of the first level from a second sub-coaching model.

According to various embodiments, in operation 1640, the health coaching system 200 (e.g., the manager model 220) may output a first health guide including the first coaching information and the second coaching information.

According to various embodiments, in operation 1650, the health coaching system 200 (e.g., the manager model 220) may determine a preferred coaching model among the first sub-coaching model and the second sub-coaching model based on feedback on the first health guide.

According to various embodiments, in operation 1660, the health coaching system 200 (e.g., the manager model 220) may provide a second coaching request query to the first sub-coaching model and the second sub-coaching model.

According to various embodiments, in operation 1670, the health coaching system 200 (e.g., the manager model 220) may obtain coaching information of a second level higher than the first level from the preferred coaching model. For example, the health coaching system 200 (e.g., the manager model 220) may obtain coaching information of the first level or may not obtain coaching information from a non-preferred coaching model. According to embodiments, the health coaching system 200 (e.g., the manager model 220) may generate a second health guide including coaching information of the at least second level.

According to various embodiments, an operating method of the electronic device 101 may include providing a first coaching request query based on the status information 201 related to a user, to the first sub-coaching model 211-1 and the second coaching sub model 211-2, obtaining the first coaching information 301 of a first level corresponding to the first coaching request query from the first sub-coaching model 211-1, obtaining the second coaching information 302 of a first level corresponding to the first coaching request query from the second sub-coaching model 211-2, displaying the first health guide 311 including the first coaching information 301 and the second coaching information 302 through the display 160 of the electronic device 101, based on a feedback about the first health guide 311, selecting at least one of the first sub-coaching model 211-1 and the second sub-coaching model 211-2 as a preferred coaching model, when providing a second coaching request query based on the status information 201 related to the user to the first sub-coaching model 211-1 and the second sub-coaching model 211-2, obtaining the third coaching information 421 of a second level higher than the first level from the preferred coaching model and obtaining the fourth coaching information 411 of the first level from another sub-coaching model except for the preferred coaching model.

According to various embodiments, the operating method of the electronic device 101 may include displaying a second health guide that at least a portion of the first health guide 311 is changed, through the display 160 based on the third coaching information and the fourth coaching information corresponding to the second coaching request query.

According to various embodiments, the operating method of the electronic device 101 may include displaying the first item 511 related to feedback for the first coaching information and a second item related to feedback for the second guide information together with the first health guide 311 and determining the feedback about the first health guide 311 based on an the input 521 for at least one of the first item 511 and the second item.

According to various embodiments, the providing of the first coaching request query to the first sub-coaching model and the second sub-coaching model may include determining preferences for the first sub-coaching model 211-1 and the second sub-coaching model 211-2, providing the first coaching request query based on first information corresponding to a first range among the status information 201 related to the user, to a model having a preference less than a specified threshold value among the first sub-coaching model 211-1 and the second sub-coaching model 211-2, and providing the first coaching request query based on second information corresponding to a second range larger than the first range among the status information 201 related to the user, to a model having a preference larger than the specified threshold value among the first sub-coaching model 211-1 and the second sub-coaching model 211-2.

According to various embodiments, the providing of the second coaching request query to the first sub-coaching model 211-1 and the second sub-coaching model 211-2 may include determining preferences for the first sub-coaching model 211-1 and the second sub-coaching model 211-2, and providing the second coaching request query based on coaching information, which is obtained from a model having a preference larger than or equal to a specified threshold value among the first sub-coaching model 211-1 and the second sub-coaching model 211-2, to a model having a preference less than a specified threshold value among the first sub-coaching model 211-1 and the second sub-coaching model 211-2.

According to various embodiments, the providing of the second coaching request query to the first sub-coaching model 211-1 and the second sub-coaching model 211-2 may include determining preferences for the first sub-coaching model 211-1 and the second sub-coaching model 211-2, and providing the second coaching request query based on coaching information, which is obtained from a model having a preference less than a specified threshold value among the first sub-coaching model 211-1 and the second sub-coaching model 211-2, to a model having a preference larger than or equal to a specified threshold value among the first sub-coaching model 211-1 and the second sub-coaching model 211-2.

According to various embodiments, the providing of the first coaching request query to the first sub-coaching model 211-1 and the second sub-coaching model 211-2, may include displaying the first coaching request query provided to the first sub-coaching model 211-1 and the second sub-coaching model 211-2 through the display 160.

According to various embodiments, the providing of the second coaching request query to the first sub-coaching model 211-1 and the second sub-coaching model 211-2, may include displaying the second coaching request query provided to the first sub-coaching model 211-1 and the second sub-coaching model 211-2 through the display 160.

According to various embodiments, the operating method of the electronic device 101 may include performing a fine-tuning to the first sub-coaching model 211-1 and the second sub-coaching model 211-2 based on feedback on the first health guide 311.

According to various embodiments, the operating method of the electronic device 101 may include obtaining an additional sub-coaching model from an external device based on feedback on the first health guide 311.

The electronic device 101 according to various embodiments disclosed in the specification may be implemented with various types of devices. The electronic device 101 may include, for example, a portable communication device (e.g., a smartphone), a computer device, a portable multimedia device, a mobile medical appliance, a camera, a wearable device, or a home appliance. The electronic device 101 according to an embodiment of this specification may not be limited to the above-described devices.

Various embodiments of the disclosure and terms used herein are not intended to limit the technical features described in the disclosure to specific embodiments, and it should be understood that the embodiments and the terms include modification, equivalent, or alternative on the corresponding embodiments described herein. With regard to description of drawings, similar or related components may be marked by similar reference marks/numerals. The singular form of the noun corresponding to an item may include one or more of items, unless interpreted otherwise in context. In the disclosure, the expressions "A or B", "at least one of A and B", "at least one of A or B", "A, B, or C", "at least one of A, B, and C", and "at least one of A, B, or C" may include any and all combinations of one or more of the associated listed items. The terms, such as "first" or "second" may be used to simply distinguish the corresponding component from the other component, but do not limit the corresponding components in other aspects (e.g., importance or order). When a component (e.g., a first component) is referred to as being "coupled with/to" or "connected to" another component (e.g., a second component) with or without the term of "operatively" or "communicatively", it may mean that a component is connectable to the other component, directly (e.g., by wire), wirelessly, or through the third component.

In various embodiments of the disclosure, the term "module" used herein may include a unit, which is implemented with hardware, software, or firmware, and may be interchangeably used with the terms "logic", "logical block", "part", or "circuit". The "module" may be a minimum unit of an integrated part or may be a minimum unit of the part for performing one or more functions or a part thereof. For example, according to an embodiment, the module may be implemented in the form of an application-specific integrated circuit (ASIC).

Various embodiments of the disclosure may be implemented with software (e.g., program 140) including one or more instructions stored in a storage medium (e.g., the embedded memory 136 or the external memory 138) readable by a machine (e.g., the electronic device 101). For example, the processor (e.g., the processor 120) of the machine (e.g., the electronic device 101) may call at least one instruction of the stored one or more instructions from a storage medium and then may execute the at least one instruction. This enables the machine to operate to perform at least one function depending on the called at least one instruction. The one or more instructions may include a code generated by a complier or a code executable by an interpreter. The machine-readable storage medium may be provided in the form of a non-transitory storage medium. Herein, 'non-transitory' just means that the storage medium is a tangible device and does not include a signal (e.g., electromagnetic waves), and this term does not distinguish between the case where data is semipermanently stored in the storage medium and the case where the data is stored temporarily.

According to an embodiment, a method according to various embodiments disclosed herein may be provided to be included in a computer program product. The computer program product may be traded between a seller and a buyer as a product. The computer program product may be distributed in the form of a machine-readable storage medium (e.g., compact disc read only memory (CD-ROM)) or may be distributed (e.g., downloaded or uploaded), through an application store (e.g., PlayStore^{™}), directly between two user devices (e.g., smartphones), or online. In the case of on-line distribution, at least part of the computer program product may be at least temporarily stored in the machine-readable storage medium such as the memory of a manufacturer's server, an application store's server, or a relay server or may be generated temporarily.

According to various embodiments, each component (e.g., a module or a program) of the above-described components may include a single entity or a plurality of entities, and some of the plurality of objects may be separately arranged on other components. According to various embodiments, one or more components of the above-described components or operations may be omitted, or one or more other components or operations may be added. Alternatively or additionally, a plurality of components (e.g., a module or a program) may be integrated into one component. In this case, the integrated component may perform one or more functions of each component of the plurality of components in the manner same as or similar to being performed by the corresponding component of the plurality of components prior to the integration. According to various embodiments, operations executed by modules, programs, or other components may be executed by a successive method, a parallel method, a repeated method, or a heuristic method. Alternatively, at least one or more of the operations may be executed in another order or may be omitted, or one or more operations may be added.

## Claims

1. An electronic device (101), comprising:
a display (160);
a memory (130) configured to store a health coaching system (200) including a first sub-coaching model (211-1), a second sub-coaching model (211-2) and a manager model (220); and
a processor (120) operatively connected with the display (160) and the memory (130) and that is configured to display a health guide through the display (160) by controlling the health coaching system (200);
under a control of the processor (120), wherein the manager model (220) is configured to:
provide a first coaching request query based on status information (201) related to a user, to the first sub-coaching model (211-1) and the second coaching sub model (211-2);
obtain first coaching information (301) of a first level corresponding to the first coaching request query from the first sub-coaching model (211-1);
obtain second coaching information (302) of the first level corresponding to the first coaching request query from the second sub-coaching model (211-2);
display a first health guide (311) including the first coaching information (301) and the second coaching information (302) through the display (160);
based on a feedback about the first health guide (311), select at least one of the first sub-coaching model (211-1) and the second sub-coaching model (211-2) as a preferred coaching model;
when providing a second coaching request query based on the status information (201) related to the user to the first sub-coaching model (211-1) and the second sub-coaching model (211-2):
obtain third coaching information (421) of a second level higher than the first level from the preferred coaching model, and
obtain fourth coaching information (411) of the first level from another sub-coaching model except for the preferred coaching model.

2. The electronic device of claim 1, wherein the manager model (220) is configured to:
based on the third coaching information and the fourth coaching information corresponding to the second coaching request query, display a second health guide that at least a portion of the first health guide (311) is changed, through the display (160).

3. The electronic device of claim 1, wherein the manager model (220) is configured to:
display a first item (511) related to feedback for the first coaching information and a second item related to feedback for the second coaching information together with the first health guide (311),
determine the feedback about the first health guide (311) based on an input (521) for at least one of the first item (511) and the second item.

4. The electronic device of claim 1, wherein the manager model (220) is configured to:
determine preferences for the first sub-coaching model (211-1) and the second sub-coaching model (211-2);
provide the first coaching request query based on first information corresponding to a first range among the status information (201) related to the user, to a model having a preference less than a specified threshold value among the first sub-coaching model (211-1) and the second sub-coaching model (211-2); and
provide the first coaching request query based on second information corresponding to a second range larger than the first range among the status information (201) related to the user, to a model having a preference larger than the specified threshold value among the first sub-coaching model (211-1) and the second sub-coaching model (211-2).

5. The electronic device of claim 1, wherein the manager model (220) is configured to:
determine preferences for the first sub-coaching model (211-1) and the second sub-coaching model (211-2); and
provide the second coaching request query to a model having a preference less than a specified threshold value among the first sub-coaching model (211-1) and the second sub-coaching model (211-2),
wherein the second coaching request query is generated based on the coaching information (301) that is obtained from a model having a preference larger than the specified threshold value among the first sub-coaching model (211-1) and the second sub-coaching model (211-2).

6. The electronic device of claim 1, wherein the manager model (220) is configured to:
determine preferences for the first sub-coaching model (211-1) and the second sub-coaching model (211-2); and
provide the second coaching request query to a model having a preference larger than a specified threshold value among the first sub-coaching model (211-1) and the second sub-coaching model (211-2),
wherein the second coaching request query is generated based on the coaching information (301) that is obtained from a model having a preference less than the specified threshold value among the first sub-coaching model (211-1) and the second sub-coaching model (211-2).

7. The electronic device of any one of claims 1, 2, 3 and 4, wherein the manager model (220) is configured to:
display the first coaching request query provided to the first sub-coaching model (211-1) and the second sub-coaching model (211-2) through the display (160).

8. The electronic device of any one of claims 1, 2, 3, 4 and 5, wherein the manager model (220) is configured to:
display the second coaching request query provided to the first sub-coaching model (211-1) and the second sub-coaching model (211-2) through the display (160).

9. The electronic device of claim 1, wherein the manager model (220) is configured to:
perform a fine-tuning to the first sub-coaching model (211-1) and the second sub-coaching model (211-2) based on feedback on the first health guide (311).

10. The electronic device of claim 1, wherein the manager model (220) is configured to:
obtain an additional sub-coaching model from an external device based on feedback on the first health guide (311).

11. An operating method of an electronic device (101), comprising:
providing a first coaching request query based on status information (201) related to a user, to a first sub-coaching model (211-1) and a second coaching sub model (211-2);
obtaining first coaching information (301) of a first level corresponding to the first coaching request query from the first sub-coaching model (211-1);
obtaining second coaching information (302) of the first level corresponding to the first coaching request query from the second sub-coaching model (211-2);
displaying a first health guide (311) including the first coaching information (301) and the second coaching information (302) through a display (160) of the electronic device (101);
based on a feedback about the first health guide (311), selecting at least one of the first sub-coaching model (211-1) and the second sub-coaching model (211-2) as a preferred coaching model;
when providing a second coaching request query based on the status information (201) related to the user to the first sub-coaching model (211-1) and the second sub-coaching model (211-2), obtaining third coaching information (421) of a second level higher than the first level from the preferred coaching model and obtaining fourth coaching information (411) of the first level from other sub-coaching model except for the preferred coaching model.

12. The operating method of claim 11, further comprising:
based on the third coaching information and the fourth coaching information, displaying a second health guide that at least a portion of the first health guide 311 is changed, through the display (160).

13. The operating method of claim 11, further comprising:
displaying a first item (511) related to feedback for the first coaching information and a second item related to feedback for the second coaching information together with the first health guide (311),
determining the feedback about the first health guide (311) based on an input (521) for at least one of the first item (511) and the second item.

14. The operating method of any one of claims 11, 12 and 13, wherein the providing the first coaching request query to a first sub-coaching model (211-1) and a second sub-coaching model (211-2) comprising:
displaying the first coaching request query provided to the first sub-coaching model (211-1) and the second sub-coaching model (211-2) through the display (160).

15. The operating method of any one of claims 11, 12, 13 and 14, wherein the providing the second coaching request query to the first sub-coaching model (211-1) and the second sub-coaching model (211-2) comprising:
displaying the second coaching request query provided to the first sub-coaching model (211-1) and the second sub-coaching model (211-2) through the display (160).
